# EUROPÄISCHE PATENTSCHRIFT

(11) **EP 1 527 042 B1**
(45) Veröffentlichungstag und Bekanntmachung des Hinweises auf die Patenterteilung: **23.07.2008**
(21) Anmeldenummer: 03766230.1
(22) Anmeldetag: 18.07.2003
(51) Int. Cl.: C07C 213/02, C07C 253/30, C07C 217/58

(54) **VERFAHREN ZUR HERSTELLUNG VON AMINOALKOXYBENZYLAMINEN UND AMINOALKOXYBENZONITRILEN ALS ZWISCHENPRODUKTE**
METHOD FOR PRODUCING AMINOALKOXY BENZYLAMINES AND AMINOALKOXY BENZONITRILES AS INTERMEDIATES
PROCEDE DE PREPARATION D'AMINOALCOXY BENZYLAMINES ET D'AMINOALCOXY BENZONITRILES EN TANT QUE PRODUITS INTERMEDIAIRES

(30) Priorität: 01.08.2002 DE 10235312
(43) Veröffentlichungstag der Anmeldung: 04.05.2005
(73) Patentinhaber: BASF SE, 67056 Ludwigshafen (DE)
(72) Erfinder: LÖBER, Oliver, 55234 Freimersheim (DE); BENISCH, Christoph, 68165 Mannheim (DE); EBEL, Klaus, 68623 Lampertheim (DE); MELDER, Johann-Peter, 67459 Böhl-Iggelheim (DE)
(86) Internationale Anmeldenummer: PCT/EP2003/007871
(87) Internationale Veröffentlichungsnummer: WO 2004/013082

(56) Entgegenhaltungen:
- EP-A- 0 758 653
- WO-A-02/076925
- BE-A- 853 789
- GB-A- 2 060 622
- US-A- 5 852 035
- US-A- 5 990 139
- LERNER, O.M. ET AL.: PHARM. CHEM. J., Bd. 7, 1969, Seiten 382-383, XP000109133 in der Anmeldung erwähnt
- FUNKE, M.A. ET AL.: BULL. SOC. CHIM. FR., Bd. 5, Nr. 12, 1945, Seiten 1050-1055, XP000116094
- DATABASE HCAPLUS ACS; 1998, XP002264256 gefunden im STN Database accession no. 129:144858 & JP 10 182459 A (OTSUKA PHARMACEUTICAL CO.) 7. Juli 1998 (1998-07-07)
- DATABASE HCAPLUS ACS; 1997, XP002264257 gefunden im STN Database accession no. 128:48243 & JP 09 291078 A (KYORIN PHARMACEUTICAL CO.) 11. November 1997 (1997-11-11)
- SHADBOLT R S ET AL: "Some Aryloxyalkylamines, N-Arylethylenediamines and Related Compounds as Anorectic Agents" JOURNAL OF MEDICINAL CHEMISTRY, AMERICAN CHEMICAL SOCIETY. WASHINGTON, US, Bd. 14, Nr. 9, 1971, Seiten 836-842, XP002084867 ISSN: 0022-2623

## Beschreibung

Die vorliegende Erfindung betrifft ein verbessertes Verfahren zur Herstellung von Aminoalkoxybenzylaminen I durch katalytische Hydrierung von Aminoalkoxybenzonitrilen II.

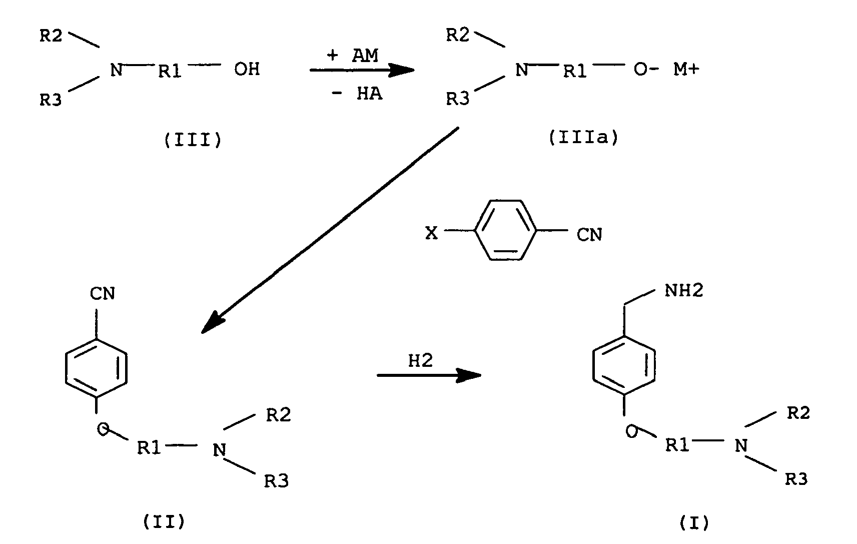

In den Verbindungen der allgemeinen Formeln I, II und III steht R¹ für C₁-C₈-Alkylen; R² und R³ bedeuten unabhängig voneinander C₁-C₈-Alkyl oder sind zu einem Ring verbunden, der zusätzlich ein Heteroatom enthalten kann; M steht für ein Alkalimetall oder ein Erdalkalimetall und A für Hydrid, C₁-C₄-Alkyl, Hydroxy oder C₁-C₄-Alkoxy; X steht für Chlor oder Brom. Im Falle der Erdalkalihydride steht A für -H₂.

Aminoalkoxybenzylamine der Formel I sind wichtige Zwischenprodukte in der Synthese von biologisch aktiven Substanzen wie beispielsweise pharmazeutischen Wirkstoffen.

O.M. Lerner und F.Yu. Rachinskii beschreiben im Author's Certificate No. 218194; Byull. Izobretenii, No. 17 (1968) die katalytische Hydrierung von 4-[2-(Dimethylamino)ethoxy]benzonitril mit Wasserstoff in Gegenwart von Raney-Nickel bei 16 bis 20°C unter Normaldruck. Die erzielten Ausbeuten an (III) von 70 bis 73 % bezogen auf (II) sind jedoch unbefriedigend.

O.M. Lerner et al. beschreiben in Pharm. Chem. J. 1969, 7, 382-383 die heterogen-katalysierte Hydrierung von 4-[2-(Dimethyl-amino)ethoxy]benzonitril mit Wasserstoff in Gegenwart von Raney-Nickel in wasserfreiem Ethanol bei Raumtemperatur und Normaldruck. Nach 10 bis 20 Stunden Hydrierdauer werden 84 % des gewünschten Produkts, bezogen auf (II), erhalten. Für eine industrielle Anwendung ist jedoch eine deutliche Verkürzung der Hydrierdauer und eine höhere Ausbeute erforderlich.

US 2879293 offenbart die Herstellung von (I) durch reduktive Aminierung von 4-[2-(Dimethylamino)ethoxy]benzaldehyd in Gegenwart von Ammoniak in Ethanol bei 80°C und 69 bar an Raney-Nickel. Die erzielten Ausbeuten werden jedoch nicht offenbart. Nachteilig ist die Verwendung des Einsatzmaterials 4-[2-(Dimethylamino)ethoxy]benzaldehyd, welches nach dem offenbarten Verfahren aus p-Hydroxybenzaldehyd und dem stark toxischen 2-Chlorethyldimethylamin hergestellt wird.

US 3193579 offenbart ebenfalls ein Verfahren zur Herstellung von 4-[2-(Dimethylamino)ethoxy]benzylamin ausgehend von 4-[2-(Dimethylamino)ethoxy]benzaldehyd in Gegenwart von Ammoniak in Ethanol bei 160°C und 21 bar an einem geträgerten PalladiumKatalysator (10 % Pd auf Calciumcarbonat). Nachteilig ist wie in US 2879293 die Verwendung von 4-[2-(Dimethylamino)ethoxy]benzaldehyd sowie der Einsatz eines teuren Edelmetallkatalysators mit hohem Edelmetallgehalt.

EP 0306827 offenbart ein Verfahren zur Herstellung von 4-[2-(Dimethylamino)ethoxy]benzylamin aus 4-[2-(Dimethylamino)ethoxy]benzaldehyd durch Oximierung mit Hydroxylaminhydrochlorid in Ethanol. Dabei wird zunächst das Benzaldoxim-Hydrochlorid erhalten, aus welchem durch Behandlung mit Kaliumcarbonat/Wasser das Benzaldoxim freigesetzt und durch Kristallisation erhalten wird (Ausbeute 95 %). Anschließend erfolgt die heterogenkatalysierte Hydrierung mit Wasserstoff an Raney-Nickel in Methanol in Gegenwart von Ammoniak bei 30°C und 49 bar. Die in der Hydrierung erzielte Ausbeute an gewünschtem Produkt wird nicht offenbart. In der zur Patentfamilie gehörenden JP 01100159 wird eine Hydrierausbeute von 93 % bezogen auf das eingesetzte Benzaldoxim erreicht. Über die bereits bei US 2879293 beschriebenen Nachteile hinaus wird das Verfahren durch die zweistufige Herstellung und die notwendige Kristallisation des Benzaldoxims aufwendig und damit unwirtschaftlich.

FR 2549828 offenbart ein mehrstufiges Verfahren zur Herstellung von 4-[2-(Dimethylamino)ethoxy]benzylamin ausgehend von Phenol. Umsetzung von Phenol mit Ethylenchlorhydrin liefert zunächst 2-Phenoxyethanol, welches mit Thionylchlorid zu 2-Phenoxyethylchlorid umgesetzt wird. Nach Destillation dieses Zwischenprodukts erfolgt die Umsetzung mit Acetamid und Paraformaldehyd und anschließender Behandlung mit Mineralsäure zu N-(2-Chlorethoxybenzyl)acetamid. Umsetzung mit Dimethylamin führt dann zu N-[2-(Dimethylamino)ethoxy]acetamid aus dem durch saure Hydrolyse das gewünschte Produkt freigesetzt wird. Das offenbarte Verfahren ist mit 6 separaten Verfahrensschritten und einer Gesamtausbeute von 50 % bezogen auf das eingesetzte Phenol nicht wirtschaftlich.

Verfahren zur Herstellung von 4-Aminoalkoxy-benznitrilen(II) sind ebenfalls bekannt:

BE 853789 offenbart ein Verfahren zur Alkoxylierung von Arylverbindungen der allgemeinen Formel X-A-Zₙ, wobei X = Halogen bedeutet, Z eine elektronenziehende Gruppe, insbesondere NO₂, darstellt und n = 1-2 ist. Die Umsetzung erfolgt mit einem Alkohol in Gegenwart von Natriumhydroxid und Tetrabutylammoniumbromid, ggf. in Chlorbenzol als Lösungsmittel unter Substitution des Halogenatoms aus X-A-Zₙ. In den angeführten Beispielen werden ausschließlich Nitroaromaten als Substrate eingesetzt.

DE 3233828 offenbart zwei Verfahren zur Herstellung von Aryloxyalkylaminen, entweder (a) durch Umsetzung eines Phenoxyalkylhalogenids der Formel Yₘ-Ar-O-(CH₂)ₙ-Hal (wobei Yₘ unter anderem auch CN bedeuten kann, m = 1-3, n = 5-12) mit einem sekundären Amin R¹R²NH oder (b) durch Umsetzung von Aminoalkylhalogeniden der Formel Hal-(CH₂)ₙ-NR¹R² mit dem Alkalisalz eines Phenols der Formel Yₘ-Ar-OH.

GB 924961 offenbart ein Verfahren zur Herstellung von (II) durch Umsetzung von p-Cyanophenol mit 2-Chlorethyldimethylamin in Toluol in Gegenwart von Natriumhydroxid. Die erzielte Ausbeute wird nicht offenbart. Nachteilig an diesem Verfahren ist die Verwendung des toxischen 2-Chlorethyldimethylamin.

Yurugi et al. beschreiben in Chem. Pharm. Bull. 1973, 21, 1641-1659 die Umsetzung von p-Cyanophenol mit 2-Chlorethyldiethylamin. Wie in GB 924961 ist die Verwendung des toxischen 2-Chlorethyldialkylamins als Nachteil zu werten.

Kmonícek et al. beschreiben in Collect. Czech. Chem. Commun. 1989, 54, 1721-1733 eine Methode zur Herstellung von (II) durch Umsetzung von 4-[2-(Dimethylamino)ethoxy]benzaldehyd mit Nitroethan durch Erhitzen mit Ammoniumacetat in Essigsäure. Nachteilig für ein industrielles Verfahren ist die Handhabung des explosionsgefährlichen Nitroethans und die niedrige Ausbeute an (II) von nur 40 % bezogen auf 4-[2-(Dimethylamino)ethoxy]-benzaldehyd.

Weiterhin beschreiben G. Uray und I. Kriessmann in Synthesis, 679-681 (1984) die Herstellung von Alkylarylethern durch Umsetzung von Alkoholen mit 4-Chlorbenzonitril in Gegenwart von Kaliumhydroxid in Dimethylsulfoxid, wobei allerdings eine Verseifung der Nitrilgruppe erfolgt.

Der vorliegenden Erfindung lag daher die Aufgabe zugrunde, unter Überwindung der Nachteile des Standes der Technik ein einfaches, wirtschaftliches Verfahren zur Herstellung von 4-[Aminoalkoxy]-benzylaminen (I) bereitzustellen, welches auf die Verwendung toxischer Reagenzien verzichtet und das gewünschte Produkt in hoher Ausbeute liefert.

Demgemäß wurde ein Verfahren zur Herstellung von 4-[Aminoalkoxy]benzylaminen (I) durch katalytische Hydrierung von 4-[Aminoalkoxy]-benzonitrilen (II) gefunden, welches dadurch gekennzeichnet ist, dass man die Hydrierung bei Drücken von 5 bis 350 bar und Temperaturen von 50 bis 250°C in Gegenwart von Ammoniak durchführt.

In den Verbindungen der allgemeinen Formeln I, II und III steht R¹ für C₁-C₈-Alkylen, bevorzugt Ethylen; R² und R³ bedeuten unabhängig voneinander C₁-C₈-Alkyl, bevorzugt Methyl oder Ethyl, oder sind zu einem gesättigten 5- oder 6-gliedrigem Ring verbunden, der zusätzlich ein Heteroatom enthalten kann, so dass NR²R³ für Pyrrolidin, Piperidin, Piperazin oder Morpholin stehen kann.

Die Erfindung betrifft insbesondere ein Verfahren zur Herstellung von 4-[2-(Dimethylamino)ethoxy]benzylamin (I) aus 4-Chlorbenzonitril über das Zwischenprodukt 4-[2-(Dimethyl-amino)ethoxy]benzonitril (II).

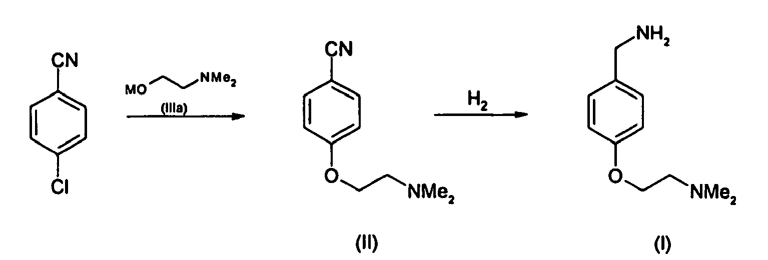

Zunächst erfolgt die Kupplung von 4-Chlorbenzonitril mit einem Alkalimetallsalz des Aminoalkohols (III) unter Erhalt von 4-[2-(Dimethylamino)ethoxy]benzonitril (II).

In einem ersten Schritt wird dabei mit Hilfe einer Base AM ein Salz des Aminoalkohols hergestellt. Als Basen werden vorteilhaft Alkalialkylverbindungen, Alkalihydride oder Alkalialkoholate wie z.B. BuLi, NaH, LiH, NaOMe oder NaOEt verwendet. Weiterhin eignen sich Erdalkalihydride wie z.B. Calciumhydrid oder Erdalkalihydroxide. Es ist auch möglich Natriumhydroxid oder Kaliumhydroxid zu verwenden, sofern Wasser aus dem Reaktionsgemisch abdestilliert wird.

Besonders bevorzugte Basen sind NaOMe und NaOEt. Auch dabei wird Methanol oder Ethanol abdestilliert. Bei Verwendung dieser Basen empfiehlt es sich, die Reaktion unter Schutzgas wie Stickstoff oder Argon durchzuführen.

Im zweiten Schritt wird das isolierte Alkalisalzes des Aminoalkohols oder vorteilhaft eine *in situ* gebildete Lösung des Alkalisalzes in einem geeigneten Lösungsmittel bei Temperaturen zwischen 100 und 140°C, bevorzugt bei Temperaturen zwischen 125 und 135°C, mit p-Chlorbenzonitril umgesetzt, wobei die Mengen an eingesetztem Alkalisalz so bemessen werden, dass ein geringer Überschuß, 1.00 bis 1.5 Äquivalente pro Äquivalent 4-Chlorbenzonitril, vorliegt. Bevorzugt wird ein geringer Überschuss eingesetzt, d.h. mindestens 1.05 Äquivalente, oder mehr.

Als Lösungsmittel für die Kupplungsreaktion werden vorteilhaft stark polare, hochsiedende, aprotische Lösungsmittel wie z.B. NMP, DMF oder DMSO benutzt. Weiterhin eignen sich Ether oder offenkettige Polyether wie beispielsweise Diphenylether, Diethylglykol, Dibutylglykol oder Dimethyldiglykol. Ebenso kann das als Reaktionspartner verwendete N,N-Dimethylaminoethanol im Überschuss eingesetzt werden und die Lösungsmittelfunktion übernehmen.

Um das Gleichgewicht möglichst vollständig auf die Seite des Salzes zu verschieben, ist darauf zu achten, den Alkohol bzw. das Wasser möglichst vollständig abzudestillieren, um die Bildung von Nebenprodukten, wie beispielsweise Methoxyderivaten, zu verhindern.

Das Wertprodukt wird aus dem Reaktionsansatz nach wässriger Aufarbeitung extraktiv gewonnen. Als Extraktionsmittel werden bevorzugt Lösungsmittel wie Ether, Ester, aliphatische und aromatische Kohlenwasserstoffe benutzt. Bevorzugt ist die Verwendung von Essigester, tert-Butylacetat, Xylol und Toluol, insbesondere Essigester und Toluol. Weiterhin eignen sich auch Mesitylen oder Decalin. Auf eine Abtrennung des bei der Reaktion ausfallenden Alkalichlorids vor der Zugabe von Wasser kann bei Verwendung von Toluol oder Xylol als Extraktionsmittel verzichtet werden. Anschließend kann die Aufarbeitung durch Destillation des Reaktionsprodukts im Vakuum bei 1 bis 20 mbar erfolgen. So liefert z.B. eine Reindestillation im Vakuum bei 5 bis 10 mbar 4-[2-(Dimethylamino)ethoxy]benzonitril (II) in Ausbeuten von bis zu 85 %. Bei Verwendung von Toluol oder Xylol kann die organische Phase auch direkt ohne Destillation der Hydrierung zugeführt werden.

In der zweiten Verfahrensstufe erfolgt die Umsetzung des 4-[Aminoalkoxy]benzonitrils (II) mit Wasserstoff an einem Katalysator unter Erhalt von 4-[Aminoalkoxy)]benzylaminen (I) bei erhöhtem Druck und erhöhter Temperatur.

Die Katalysatoren, die in dem erfindungsgemäßen Verfahren eingesetzt werden, enthalten in der aktiven Katalysatormasse 2 bis 100 Gew.-% mindestens eines Elements oder mindestens eine Verbindung eines Elements aus der Gruppe VIII des Periodensystems, also aus der Gruppe bestehend aus Fe, Ru, Os, Co, Rh, Ir, Ni, Pd, Pt. Besonders bevorzugt sind Cobalt und Nickel, insbesondere Raney-Cobalt und Raney-Nickel.

Die Katalysatoren können als Vollkontakt oder in geträgerter Form eingesetzt werden. Bei Einsatz von geträgerten Katalysatoren beträgt der Anteil des Trägers an der Gesamtmasse des Katalysators (Aktivmasse+Träger) im allgemeinen 10 bis 98 Gew.-%.

Als Träger können alle bekannten geeigneten Träger verwendet werden, beispielsweise Aktivkohle, Siliciumcarbid oder Metalloxide. Von den Metalloxiden werden vorzugsweise Aluminiumoxid, Siliciumdioxid, Titandioxid, Zirkoniumdioxid, Zinkoxid, Magnesiumoxid oder deren Gemische verwendet, die gegebenenfalls mit Alkali- und/oder Erdalkalioxiden dotiert sein können. Die Träger können in beliebiger Form eingesetzt werden, beispielsweise als Extrudate (in Form von Strängen), Pellets, Tabletten, Monolithe, Gewebe, Gestricke oder pulverförmig. Die geträgerten Katalysatoren können nach den allgemein bekannten Verfahren hergestellt werden.

Das erfindungsgemäße Verfahren wird bei Temperaturen von 50 bis 250°C, vorzugsweise 60 bis 110°C und Drücken von 5 bis 350 bar, vorzugsweise 5 bis 200 bar, besonders bevorzugt 10 bis 80 bar, kontinuierlich oder bevorzugt diskontinuierlich in Druckapparaturen wie Rohrreaktoren oder bevorzugt in einem Autoklaven durchgeführt. Der Druck ist dabei vorzugsweise der Wasserstoffdruck im Autoklaven.

Das erfindungsgemäße Verfahren lässt sich lösungsmittelfrei oder bevorzugt in Lösungsmitteln wie Alkoholen, Ethern, cyclischen Ethern, aliphatischen Kohlenwasserstoffen oder aromatischen Kohlenwasserstoffen durchführen. Bevorzugt ist die Verwendung von Methanol, Tetrahydrofuran oder Toluol, insbesondere Methanol oder Toluol. Im Lösungsmittel kann dabei das eingesetzte Nitril (II) gelöst sein. Das Lösungsmittel kann auch getrennt dem Reaktor an beliebiger Stelle zugeführt werden. Bei Verwendung eines Lösungsmittels beträgt die Konzentration an Nitril (II) im Lösungsmittel 5 bis 80 Gew.-% (bezogen auf die Summe von Nitril und Lösungsmittel), bevorzugt 5 bis 50 Gew.-%, besonders bevorzugt 20 bis 50 Gew.-%.

Die erfindungsgemäße Hydrierung erfolgt in Gegenwart von Ammoniak. Der Ammoniakgehalt beträgt im allgemeinen von 1 bis 50, bevorzugt 2 bis 20 Mol Ammoniak pro Mol des zu hydrierenden Nitrils.

Die Reaktionsdauer kann über den Wasserstofffluss bemessen werden, wobei die Reaktion als abgeschlossen gilt, wenn kein Wasserstofffluss mehr stattfindet, d.h. dass man keinen Wasserstoff mehr nachpressen muss, um den Druck konstant zu halten. Weiterhin kann der Reaktionsverlauf durch gaschromatographische Analyse von Proben des Reaktionsgemischs überprüft werden. Eine weitere Möglichkeit bieten Infrarotmessungen, wobei der Reaktionsverlauf über das verschwinden der Nitrilbande verfolgt werden kann.

Das erfindungsgemäße Verfahren ermöglicht auf einfache und kostengünstige Weise die Herstellung von Aminoalkoxybenzylaminen sowie des Zwischenproduktes II in guten Ausbeuten. Überraschenderweise tritt keine nennenswerte Kernhydrierung des Aromaten auf. Überraschend war auch, dass der Katalysator mehrmals in die Reaktion zurückgeführt werden kann, obwohl dies bei Nitrilhydrierungen normalerweise wegen einer Desaktivierung des Katalysators problematisch sein kann.

Vorteilhaft ist auch, dass bei der Herstellung des Zwischenprodukts II eine Verseifung der Nitrilgruppe vermieden wird.

Die Erfindung wird nun in den nachstehenden Beispielen näher erläutert.

### Beispiel 1

Unter Rühren bei RT wurden 660 ml N,N-Dimethylaminoethanol in einem Glas-Vierhalskolben unter Argonatmosphäre vorgelegt. In 45 min wurden 144,4 g 30%ige NaOMe-Lösung (in MeOH) zugetropft. Der Kolbeninhalt wurde für 3 h auf 130°C erwärmt und dabei MeOH abdestilliert. Danach wurde bei RT eine Lösung von 91,8 g p-Chlorbenzonitril in 460 ml Dimethylaminoethanol innerhalb von 45 min zugetropft. Anschließend wurde der Kolbeninhalt 4 h bei 130°C gerührt. Dann wurde der angefallene Feststoff (überwiegend NaCl) bei RT abgesaugt und das Filtrat am Rotationsverdampfer bei 70°C und 15 mbar eingeengt. Daraufhin wurde der Rückstand mit 200 ml Wasser und 400 ml Essigester extrahiert. Die wässrige Phase wurde nochmals mit 200 ml Essigester extrahiert und die vereinigten organischen Phasen über MgSO₄ getrocknet. Das Lösungsmittel wurde dann am Rotationsverdampfer abgezogen und der Rückstand über eine Kolonne destilliert. Bei etwa 160°C und 5 mbar erhielt man 4-[2-(Dimethylamino)ethoxy]benzonitril in einer Ausbeute von 69,2 % (89,2 g) mit einer GC-Reinheit von > 97 % (Flächenprozent).

### Beispiel 2

Unter Rühren wurden bei RT in einem 1000 ml-Glas-Vierhalskolben unter Argonatmosphäre 600 g Dimethylaminoethanol vorgelegt. In 15 min wurden 144,4 g 30%ige Natrium-Methylat-Lösung zugetropft. Der Kolbeninhalt wurde langsam erwärmt. Bei 100°C Sumpftemperatur begann die Lösung zu sieden (70°C Kopftemperatur). In 2 h wurde nun auf 135°C erwärmt, dabei stieg die Kopftemperatur auf 125°C an. Dann wurde 1 h bei 135°C gerührt. Danach wurden 91,8 g p-Chlorbenzonitril als Feststoff bei 120°C zugegeben. Anschließend wurde der Kolbeninhalt 4 h bei 125°C gerührt. Das Filtrat wurde am Rotationsverdampfer bei 70°C und 20 mbar eingeengt. Dann wurde der Rückstand bei RT mit 300 ml Wasser versetzt und mit 300 ml Toluol extrahiert. Die so erhaltene toluolische Lösung des Benzonitrils wurde direkt zum Benzylamin weiterhydriert (vgl. Beispiel 6).

Bei der destillativen Aufarbeitung eines analogen Ansatzes erhielt man 4-[2-(Dimethylamino)ethoxy]benzonitril in einer Ausbeute von 80,3 % (102,9 g) mit einer GC-Reinheit von mehr als 98 % (Flächenprozent).

### Beispiel 3

In einen 270-ml-Druckautoklaven mit Begasungsrüher wurden 15 g 4-[2-(Dimethylamino)ethoxy]benzonitril (II), 85 g Methanol und 3 g Raney-Nickel eingebaut, der Autoklav verschlossen und mit Stickstoff inertisiert. Anschließend wurden 3 g Ammoniak aufgepresst und auf 80°C Innentemperatur erhitzt. Bei dieser Innentemperatur wurde Wasserstoff auf 65 bar aufgepresst. Nach 34 min war die Wasserstoffaufnahme beendet. Es wurde noch eine Stunde nachgerührt, dann auf Raumtemperatur abgekühlt, entspannt und der ammoniakfreie Reaktoraustrag mittels Gaschromatographie untersucht. Der Umsatz an (II) betrug 99,5 %, die Selektivität zu 4-[2-(Dimethylamino)ethoxy]benzylamin betrug 95,8 %.

### Beispiel 4

Der ausgebaute Katalysator aus Beispiel 3 wurde mit 100 ml Methanol gewaschen und erneut in die Reaktion eingesetzt. Die Durchführung erfolgte wie in Beispiel 3 beschrieben. Nach der Reaktion wurde der Reaktoraustrag wie in Beispiel 3 gaschromatographisch analysiert und der Katalysator mit 100 ml Methanol gewaschen und erneut in die Reaktion eingesetzt. Die Katalysatorrückführung wurde fünfmal wiederholt. Die Ergebnisse sind in Tabelle 1 zusammengestellt.

**Tabelle 1**

| Rückführung Nr. | Wasserstoffaufnahmezeit [min] | Umsatz [%] | Selektivität [%] |
|---|---|---|---|
| | | | |
| 1 | 34 | 99,4 | 95,8 |
| 2 | 34 | 99,3 | 96,8 |
| 3 | 38 | 99,1 | 97,3 |
| 4 | 48 | 99,2 | 97,0 |
| 5 | 58 | 99,1 | 97,7 |

### Beispiel 5

In einen 270-ml-Druckautoklaven mit Begasungsrüher wurden 15 g 4-[2-(Dimethylamino)ethoxy]benzonitril (II), 85 g Methanol und 3 g Raney-Nickel eingebaut, der Autoklav verschlossen und mit Stickstoff inertisiert. Anschließend wurde eine bestimmte Menge Ammoniak (vgl. Tabelle 2) aufgepresst und auf 80°C Innentemperatur erhitzt. Bei dieser Innentemperatur wurde Wasserstoff auf 65 bar aufgepresst. Nach Ende der Wasserstoffaufnahme wurde noch eine Stunde nachgerührt, dann auf Raumtemperatur abgekühlt, entspannt und der ammoniakfreie Reaktoraustrag mittels Gaschromatographie untersucht. Die Wasserstoffaufnahmezeiten, Umsätze und Selektivitäten sind Tabelle 2 zu entnehmen.

**Tabelle 2**

| Beispiel Nr. | Ammoniakmenge [g] | Wasserstoff-aufnahmezeit [min] | Umsatz [%] | Selektivität [%] |
|---|---|---|---|---|
| | | | | |
| 5a | 0 | 70 | 99,5 | 81,7 |
| 5b | 3 | 34 | 99,5 | 95,8 |
| 5c | 10 | 13 | 99,9 | 97,6 |

### Beispiel 6

In einen 270-ml-Druckautoklaven mit Begasungsrüher wurden 150 g des toluolhaltigen Reaktionsaustrags aus Beispiel 2 und 4 g Raney-Nickel eingebaut, der Autoklav verschlossen und mit Stickstoff inertisiert. Anschließend wurden 15 g Ammoniak aufgepresst und auf 80°C Innentemperatur erhitzt. Bei dieser Innentemperatur wurde Wasserstoff auf 65 bar aufgepresst. Nach 20 min war die Wasserstoffaufnahme beendet. Es wurde noch eine Stunde nachgerührt, dann auf Raumtemperatur abgekühlt, entspannt und der ammoniakfreie Reaktoraustrag mittels Gaschromatographie untersucht. Der Umsatz an (II) betrug 99,8 %, die Selektivität zu 4-[2-(Dimethylamino)ethoxy]benzylamin betrug 96,0 %.

Zur Isolierung des Wertprodukts wurde das Lösungsmittel am Rotationsverdampfer entfernt und das Rohprodukt über eine 20 cm-Vigreuxkolonne bei 10 mbar fraktioniert destilliert. Die isolierte Ausbeute an 4-[2-(Dimethylamino)ethoxy]benzylamin bezogen auf das eingesetzte 4-Chlorbenzonitril betrug 82 %.

## Patentansprüche

1. Verfahren zur Herstellung von 4-[Aminoalkoxy]benzylaminen der allgemeinen Formel (I) 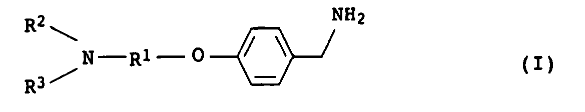 durch katalytische Hydrierung von 4-[Aminoalkoxy]-benzonitrilen der allgemeinen Formel (II), 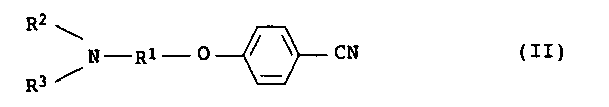 wobei in den Verbindungen der allgemeinen Formeln I und II R¹ für C₁-C₈-Alkylen steht, R² und R³ unabhängig voneinander C₁-C₈-Alkyl bedeuten oder zu einem Ring verbunden sind, der zusätzlich ein Heteroatom enthalten kann, **dadurch gekennzeichnet, dass** man die Hydrierung bei Drücken von 5 bis 350 bar und Temperaturen von 50 bis 250°C in Gegenwart von Ammoniak durchführt.

2. Verfahren nach Anspruch 1, **dadurch gekennzeichnet, dass** man die Hydrierung bei Drücken von 5 bis 200 bar durchführt.

3. Verfahren nach einem der Ansprüche 1 oder 2, **dadurch gekennzeichnet, dass** man die Hydrierung bei Temperaturen von 60 bis 110°C durchführt.

4. Verfahren nach einem der Ansprüche 1 bis 3, **dadurch gekennzeichnet, dass** man die Hydrierung in Gegenwart eines organischen Lösungsmittels durchführt.

5. Verfahren nach einem der Ansprüche 1 bis 4, **dadurch gekennzeichnet, dass** man die Hydrierung in Gegenwart von Raney-Nikkel oder Raney-Cobalt durchführt.

6. Verfahren nach einem der Ansprüche 1 bis 5, **dadurch gekennzeichnet, dass** man das Zwischenprodukt (II) durch Umsetzung eines 4-Halogenbenzonitrils mit einem Alkalisalz eines Aminoalkohols der allgemeinen Formel (III) 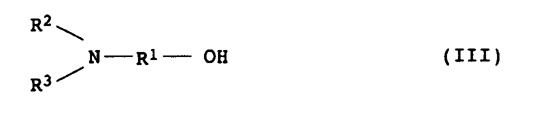 worin R¹, R² und R³ die oben stehende Bedeutung haben, erhält.

7. Verfahren nach Anspruch 6, **dadurch gekennzeichnet, dass** man das Alkalisalz des Aminoalkohols (III) durch Umsetzung mit einer Base AM, wobei M für ein Alkalimetall oder ein Erdalkalimetall steht und A für Hydrid, C1-C4-Alkyl, Hydroxy oder C₁-C₄-Alkoxy, erhält.

8. Verfahren nach einem der Ansprüche 1 bis 7, **dadurch gekennzeichnet, dass** R¹ für Ethylen und R² und R³ für Methyl stehen

9. Verfahren nach Anspruch 7, **dadurch gekennzeichnet, dass** man als Base AM Natriummethanolat oder Natriumethanolat einsetzt.

10. Verfahren nach Anspruch 9, **dadurch gekennzeichnet, dass** man Methanol oder Ethanol aus dem Reaktionsgemisch abdestilliert.

11. Verfahren nach einem der Ansprüche 6 bis 10, **dadurch gekennzeichnet, dass** man die Umsetzung des Alkalisalzes in Gegenwart eines Lösungsmittels durchführt.

12. Verfahren nach einem der Ansprüche 6 bis 11, **dadurch gekennzeichnet, dass** man die Umsetzung des Alkalisalzes bei Temperaturen von 100 bis 140°C durchführt.

13. Verfahren nach einem der Ansprüche 6 bis 12, **dadurch gekennzeichnet, dass** die Menge des Alkalisalzes 1.00 bis 1.5. Äquivalenten, bezogen auf 4-Halogenbenzonitril, beträgt.

## Claims

1. A process for preparing 4-[aminoalkoxy]benzylamines of the general formula (I) 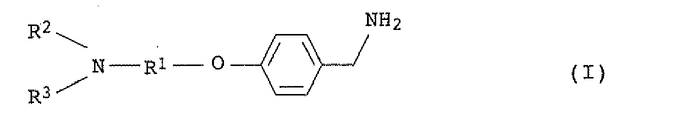 by catalytically hydrogenating 4-[aminoalkoxy]benzonitriles of the general formula (II) 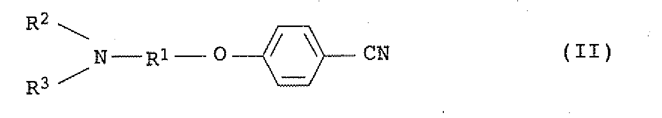 where, in the compounds of the general formulae I and II, R¹ is C₁-C₈-alkylene, R² and R³ are each independently C₁-C₈-alkyl or are joined to give a ring which may additionally comprise a heteroatom, which comprises carrying out the hydrogenation at pressures of from 5 to 350 bar and temperatures of from 50 to 250°C in the presence of ammonia.

2. The process according to claim 1, wherein the hydrogenation is carried out at pressures of from 5 to 200 bar.

3. The process according to either of claims 1 or 2, wherein the hydrogenation is carried out at temperatures of from 60 to 110°C.

4. The process according to any of claims 1 to 3, wherein the hydrogenation is carried out in the presence of an organic solvent.

5. The process according to any of claims 1 to 4, wherein the hydrogenation is carried out in the presence of Raney nickel or Raney cobalt.

6. The process according to any of claims 1 to 5, wherein the intermediate (II) is obtained by reacting a 4-halobenzonitrile with an alkali metal salt of an aminoalcohol of the general formula (III) 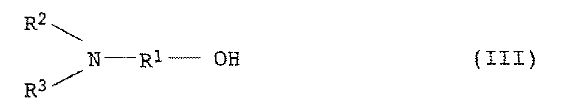 where R¹, R² and R³ are each as defined above.

7. The process according to claim 6, wherein the alkali metal salt of the aminoalcohol (III) is obtained by reaction with a base AM where M is an alkali metal or an alkaline earth metal and A is hydride, C₁-C₄-alkyl, hydroxyl or C₁-C₄-alkoxy.

8. The process according to any of claims 1 to 7, wherein R¹ is ethylene and R² and R³ are each methyl.

9. The process according to claim 7, wherein the base AM used is sodium methoxide or sodium ethoxide.

10. The process according to claim 9, wherein methanol or ethanol is distilled out of the reaction mixture.

11. The process according to any of claims 6 to 10, wherein the reaction of the alkali metal salt is carried out in the presence of a solvent.

12. The process according to any of claims 6 to 11, wherein the reaction of the alkali metal salt is carried out at temperatures of from 100 to 140°C.

13. The process according to any of claims 6 to 12, wherein the amount of the alkali metal salt is from 1.00 to 1.5 equivalents, based on 4-halobenzonitrile.

## Revendications

1. Procédé de préparation de 4-[aminoalcoxy]benzylamines de formule générale (I) : 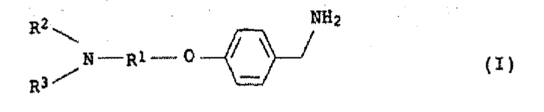 par hydrogénation catalytique de 4-[aminoalcoxy]-benzonitriles de formule générale (II), 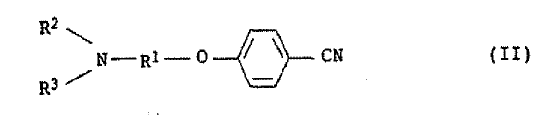 dans les composés des formules générales I et II, R¹ représentant de l'alkylène en C₁-C₈, R² et R³ représentant indépendamment l'un de l'autre de l'alkyle en C₁-C₈ ou étant liés à un cycle, qui peut comprendre de manière supplémentaire un hétéroatome, **caractérisé en ce que** l'hydrogénation est mise en oeuvre à des pressions de 5 à 350 bars et des températures de 50 à 250 °C en présence d'ammoniac.

2. Procédé selon la revendication 1, **caractérisé en ce que** l'hydrogénation est mise en oeuvre à des pressions de 5 à 200 bars.

3. Procédé selon la revendication 1 ou 2, **caractérisé en ce que** l'hydrogénation est mise en oeuvre à des températures de 60 à 110 °C.

4. Procédé selon l'une quelconque des revendications 1 à 3, **caractérisé en ce que** l'hydrogénation est mise en oeuvre en présence d'un solvant organique.

5. Procédé selon l'une quelconque des revendications 1 à 4, **caractérisé en ce que** l'hydrogénation est mise en oeuvre en présence de nickel de Raney ou de cobalt de Raney.

6. Procédé selon l'une quelconque des revendications 1 à 5, **caractérisé en ce que** le produit intermédiaire (II) est obtenu en amenant à réagir un 4-halogénobenzonitrile avec un sel alcalin d'un alcool amino de formule générale (III) : 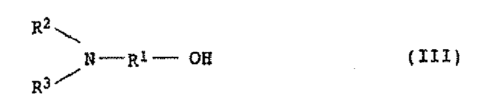 dans laquelle R¹, R² et R³ ont la signification indiquée ci-dessus.

7. Procédé selon la revendication 6, **caractérisé en ce que** le sel alcalin de l'alcool amino (III) est obtenu par réaction avec une base AM, M représentant un métal alcalin ou un métal alcalino-terreux et A de l'hydrure, de l'alkyle en C₁-C₄, de l'hydroxy ou de l'alcoxy en C₁-C₄.

8. Procédé selon l'une quelconque des revendications 1 à 7, **caractérisé en ce que** R¹ représente de l'éthylène et R² et R³ du méthyle.

9. Procédé selon la revendication 7, **caractérisé en ce que** du méthanolate de sodium ou de l'éthanolate de sodium est utilisé en tant que base AM.

10. Procédé selon la revendication 9, **caractérisé en ce que** du méthanol ou de l'éthanol est éliminé du mélange de réaction par distillation.

11. Procédé selon l'une quelconque des revendications 6 à 10, **caractérisé en ce que** la réaction du sel alcalin est mise en oeuvre en présence d'un solvant.

12. Procédé selon l'une quelconque des revendications 6 à 11, **caractérisé en ce que** la réaction du sel alcalin est mise en oeuvre à des températures de 100 à 140 °C.

13. Procédé selon l'une quelconque des revendications 6 à 12, **caractérisé en ce que** la quantité du sel alcalin est de 1,00 à 1,5 équivalent, par rapport au 4-halogénobenzonitrile.
